# EUROPEAN PATENT SPECIFICATION

(11) **EP 2 887 319 B1**
(45) Date of publication and mention of the grant of the patent: **15.04.2020**
(21) Application number: 14164705.7
(22) Date of filing: 15.04.2014
(51) Int. Cl.: G06T 7/62

(54) **Method and apparatus for indicating point whose location has been adjusted based on type of caliper in medical image**
Verfahren und Vorrichtung zur Anzeige eines Punktes, dessen Ort auf der Basis des Tasters in medizinischen Bildern eingestellt wurde
Procédé et appareil permettant d'indiquer un point dont l'emplacement a été ajusté sur la base du type d'étrier dans une image médicale

(30) Priority: 20.12.2013 KR 20130160581
(43) Date of publication of application: 24.06.2015
(73) Proprietor: Samsung Medison Co., Ltd., Gangwon-do 250-870 (KR)
(72) Inventor: Yoo, Jun-sang, Gangwon-do (KR)
(74) Representative: Schmid, Wolfgang

(56) References cited:
- EP-A1- 1 911 402
- CN-A- 102 626 326
- JP-A- 2010 240 198
- US-A- 5 605 155
- WENDELHAG I ET AL: "A new automated computerized analyzing system simplifies readings and reduces the variability in ultrasound measurement of intima-media thickness", STROKE, LIPPINCOTT WILLIAMS & WILKINS, US, vol. 28, no. 11, 1 November 1997 (1997-11-01), pages 2195-2200, XP009126865,
- STEFAN PUCHNER ET AL: "Assessment of intima-media thickness of carotid arteries: evaluation of an automated computer software", NEURORADIOLOGY ; A JOURNAL DEVOTED TO NEUROIMAGING AND INTERVENTIONAL NEURORADIOLOGY OFFICIAL JOURNAL OF THE EUROPEAN SOCIETY OF NEURORADIOLOGY ORGAN OF THE JAPANESE NEURORADIOLOGICAL SOCIETY, SPRINGER, BERLIN, DE, vol. 50, no. 10, 12 June 2008 (2008-06-12) , pages 849-853, XP019653675,
- JARDIM S M G V B ET AL: "Segmentation of fetal ultrasound images", ULTRASOUND IN MEDICINE AND BIOLOGY, NEW YORK, NY, US, vol. 31, no. 2, 1 February 2005 (2005-02-01), pages 243-250, XP027605567, [retrieved on 2005-02-01]
- OLABARRIAGA S D ET AL: "Interaction in the segmentation of medical images: A survey", MEDICAL IMAGE ANALYSIS, OXFORD UNIVERSITY PRESS, OXOFRD, GB, vol. 5, no. 2, 13 June 2001 (2001-06-13), pages 127-142, XP002463194,
- Vascular ET AL: "Vascular Health and Risk Management", Health and Risk Management, 1 January 2009 (2009-01-01), pages 811-817, XP055274143, Retrieved from the Internet: URL:http://www.ncbi.nlm.nih.gov/pmc/articl es/PMC2754094/pdf/vhrm-5-811.pdf

## Description

### BACKGROUND

### 1. Field

One or more embodiments of the present invention relate to a method of and an apparatus for indicating a location-adjusted point in a medical image in which the position of the point is adjusted based on a type of caliper to be used to measure an object.

### 2. Description of the Related Art

Medical diagnostic apparatuses include an ultrasound diagnostic apparatus, an x-ray photographing apparatus, a computerized tomography (CT) apparatus, a magnetic resonance imaging (MRI) photographing apparatus, and the like.

The ultrasound diagnostic apparatus transmits an ultrasound signal to a predetermined part of a body from the body surface of the body being an object and uses information of the ultrasound signal reflected from an organ of the body to obtain a tomogram of a soft tissue or an image of a blood stream.

Such an ultrasound diagnostic apparatus has an advantage of indicating information with respect to the object in real time. Also, since the ultrasound diagnostic apparatus has a merit of high stability without an exposure to x-ray radiation or the like, it is widely used along with other image diagnostic apparatuses, such as an x-ray diagnostic apparatus, a CT scanner, an MRI apparatus, and a nuclear medicine diagnostic apparatus.

The x-ray photographing apparatus is used as a radiographic photographing apparatus for a medical industry.

An x-ray that is an electromagnetic wave has a nature of penetrating an object, thereby being widely used in medical equipment for photographing the inside of a living body or a nondestructive inspection apparatus of a general industry.

Basic principles of the photographing apparatus using x-rays include making the x-rays released from x-ray tubes (or x-ray sources) pass through an object, detecting a strength difference of the x-ray that has passed through the object by a detector, and understanding an internal structure of the object. The photographing apparatus using the x-ray has an advantage of easily grasping the internal structure of the object by using a principle that a transmission rate of the x-ray varies according to a density of an object and atomic numbers of atoms forming an object. If a wavelength of an x-ray is short, a transmission rate increases and a brightness value of an image increases.

The photographing apparatus using the x-ray may generally include an x-ray source, an x-ray detector, and an imaging processing apparatus. The x-ray source irradiates a light source of x-ray under a predetermined condition for x-ray irradiation, and the x-ray detector obtains and sends image data based on an x-ray that has passed through an object to the imaging processing apparatus. The imaging processing apparatus may process the image data and provide an image of the object to an indication unit.

The CT apparatus is also used as the radiographic photographing apparatus for the medical industry. The CT apparatus may provide a tomogram of an object, thereby having an advantage of indicating an internal structure of the object (for example, organs such as kidneys and lungs) in a non-overlapping way, compared to a general x-ray photographing apparatus.

The CT apparatus may provide a relatively accurate tomogram with respect to the object by obtaining and processing image data of a thickness of 2 mm or less in tens to hundreds of times per second. That is, the CT apparatus has an advantage of generating a two-dimensional or a three-dimensional image of the internal structure of the object by using a plurality of pieces of two-dimensional x-ray image data photographed around a rotation shaft.

There is also the MRI photographing apparatus used as a medical diagnostic apparatus.

The MRI is a technology that obtains an image with respect to an object by locating the object in a large magnetic space generating a magnetic field, generating a radio frequency pulse to resonate a proton included in the object, and measuring a difference between signals generated from tissues included in the object and reconstructing the image of the object from the difference, via a computer.

The MRI has higher resolution and contrast than other imaging methods using an ultrasound wave or the like, and enables providing a deep organ image and three-dimensional information in real time. In addition, the MRI is harmless to a human body since it has no exposure to radioactivity, and may obtain axial, sagittal, and coronal images without shifting a location of the object.

Wendelhag et al., "A new automated computerized analyzing system simplifies readings and reduces the variability in ultrasound measurement of intima-media thickness", Stroke, Lippincott Williams & Wilkins, US, vol. 28, no. 11, 1 November 1997, pages 2195-2200, discloses an automated system for obtaining measurements in ultrasound images allowing for an interactive modification by a human operator and using local measurements of intensity, intensity gradient, and boundary continuities in a procedure for determining the optimal location of the vessel interfaces.

### SUMMARY

One or more embodiments of the present invention include a method of and an apparatus for indicating in a medical image a point whose location has been adjusted based on a type of caliper to be used to measure an object.

Additional aspects will be set forth in part in the description which follows and, in part, will be apparent from the description, or may be learned by practice of the presented embodiments.

The invention is defined by claim 1. Regarding the apparatus for processing a medical image the invention is defined by claim 5.

According to one or more embodiments of the present invention, a method of indicating in a medical image a point whose location has been adjusted based on a type of caliper to be used to measure an object includes: obtaining the medical image with respect to the object; setting a measurement item and the type of caliper with respect to the object; selecting a point in the medical image based on an external input; adjusting a location of the selected point based on the type of caliper that has been set; and indicating the location-adjusted point in the medical image.

The type of caliper may be determined so as to select at least one of an inner point, an outer point, and a middle point of a boundary of the object according to the measurement item.

The selecting of the point in the medical image based on the external input may include determining an order of selecting the point according to the measurement item that has been set, and selecting at least one point according to the determined order.

The adjusting of the location of the selected point based on the type of caliper that has been set includes: obtaining location information of the selected point; extracting a point adjacent to the selected point and corresponding to the type of caliper that has been set; and adjusting a location of the selected point according to location information of the extracted point.

The indicating of the location-adjusted point in the medical image may include indicating the adjusted point, by shifting an identifier indicating the selected point.

The indicating of the location-adjusted point in the medical image may include indicating an identifier indicating the location-adjusted point, together with an identifier indicating the selected point.

According to one or more embodiments of the present invention, an apparatus for indicating in a medical image a point whose location has been adjusted based on a type of caliper to be used to measure an object includes: an ultrasound image obtaining unit that obtains the medical image with respect to the object; a setting unit that sets a measurement item and the type of caliper with respect to the object; a selection unit that selects a point in the medical image based on an external input; an adjusting unit that adjusts a location of the selected point based on the type of caliper that has been set; and an indication unit that indicates the adjusted point in the medical image.

The type of caliper may be determined so as to select at least one of an inner point, an outer point, and a middle point of a boundary of the object according to the measurement item.

The selection unit may further include an order determination unit that determines an order of selecting the point according to the measurement item that has been set, and the selection unit may select at least one point according to the determined order.

The adjusting unit includes: a location information obtaining unit that obtains location information of the selected point; a point extraction unit that extracts a point adjacent to the selected point and corresponding to the type of caliper that has been set; and a location adjusting unit that adjusts a location of the selected point according to location information of the extracted point.

The indication unit may indicate the adjusted point, by shifting an identifier indicating the selected point.

The indication unit may indicate an identifier indicating the location-adjusted point, together with an identifier indicating the selected point.

According to one or more embodiments of the present invention, a non-transitory computer-readable medium has embodied thereon a computer program for executing the method described above.

### BRIEF DESCRIPTION OF THE DRAWINGS

These and/or other aspects will become apparent and more readily appreciated from the following description of the embodiments, taken in conjunction with the accompanying drawings in which:
FIG. 1 is a flowchart illustrating a method of indicating in a medical image a point whose location has been adjusted based on a type of caliper to be used to measure an object, according to an embodiment of the present invention;
FIGS. 2A through 2E illustrate an example of a type of caliper that has been set according to a measurement item;
FIG. 3A illustrates an example of a point whose location has been adjusted in correspondence to a type of caliper, the point indicated in an ultrasound image, according to an embodiment of the present invention;
FIG. 3B illustrates an example of indicating a point designated according to a touching input of a user by adjusting the point according to a type of caliper, according to an embodiment of the present invention;
FIG. 3C illustrates an example of a point whose location has been adjusted in correspondence to a type of caliper, the point indicated in an x-ray image, according to an embodiment of the present invention;
FIG. 3D illustrates an example of a point whose location has been adjusted in correspondence to a type of caliper, the point indicated in a computerized tomography (CT) image, according to an embodiment of the present invention;
FIG. 3E illustrates an example of a point whose location has been adjusted in correspondence to a type of caliper, the point indicated in a magnetic resonance imaging (MRI) image, according to an embodiment of the present invention;
FIG. 4 illustrates a process of extracting a point corresponding to a type of caliper, according to an embodiment of the present invention;
FIG. 5 illustrates an example of a point whose location has been adjusted in correspondence to a type of caliper, the point indicated, according to an embodiment of the present invention;
FIG. 6 illustrates an example of indicating a point whose location has been adjusted in correspondence to a type of caliper, according to another embodiment of the present invention; and
FIG. 7 is a block diagram of an apparatus for indicating in a medical image a point whose location has been adjusted based on a type of caliper to be used to measure an object, according to an embodiment of the present invention.

### DETAILED DESCRIPTION

Hereinafter, the terms used in the specification will be briefly described, and then the present invention will be described in detail.

The terms used in this specification are those general terms currently widely used in the art in consideration of functions in regard to the present invention, but the terms may vary according to the intention of those of ordinary skill in the art, precedents, or new technology in the art. Also, specified terms may be selected by the applicant, and in this case, the detailed meaning thereof will be described in the detailed description of the invention. Thus, the terms used in the specification should be understood not as simple names but based on the meaning of the terms and the overall description of the invention.

Throughout the specification, it will also be understood that when a component "includes" an element, unless there is another opposite description thereto, it should be understood that the component does not exclude another element but may further include another element. In addition, terms such as "... unit", "... module", or the like refer to units that perform at least one function or operation, and the units may be implemented as hardware or software or as a combination of hardware and software.

As used herein, the term "medical image" refers to multi-dimensional data composed of discrete image elements (e.g., pixels for 2D images and voxels for 3D images). For example, the medical image may include an image with respect to an object, collected by an ultrasound wave, an x-ray, computer tomography (CT), magnetic resonance imaging (MRI), or any other medical imaging system known to one of skill in the art. The object may refer to a part of a body. For example, the object may include a liver, a heart, a uterus, a brain, a breast, organs in abdomen, or a fetus.

Furthermore, in the present specification, "user" refers to a medical professional, such as a doctor, a nurse, a medical laboratory technologist, a medical imaging technologist, or a sonographer, but the user is not limited thereto.

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

The user identifies a growth condition of a fetus, and measures a length or the like of a part of interest in a standard image of anatomical parts to determine whether there is a malformation. When a caliper is used to measure the part of interest, the user performs the measurement by setting a type of caliper in accordance with measurement guidelines provided by specific organizations, such as hospitals, home and foreign societies, or home and foreign ultrasound organizations. For example, when a difference of an inner portion and an outer portion of a boundary line is clear as in a biparental diameter (BPD), it may be possible to accurately designate a measurement point. However, it may be difficult depending on the level skill of the user. Moreover, it may take a lot of time to accurately designate the measurement point.

Furthermore, the user may feel the measurement process inconvenient as the user needs to remember measurement guidelines predetermined according to measurement items.

According to the one or more of the above embodiments of the present invention, a measurement point may be automatically adjusted and designated according to a measurement item and a type of caliper that are set, when the measurement item and the type of caliper are set. Thus, the measurement point may be accurately designated regardless of the skill level of the user, and, as the time of designating the measurement point decreases, the overall inspection time of the object may be reduced.

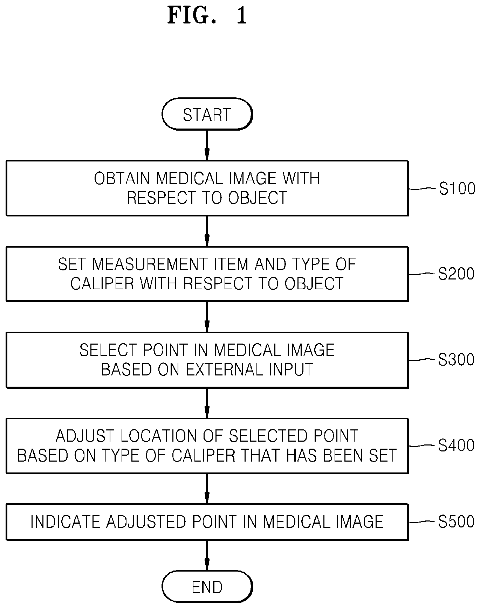

FIG. 1 is a flowchart illustrating a method of indicating in a medical image a point whose location has been adjusted based on a type of caliper to be used to measure an object, according to an embodiment of the present invention.

The method of indicating in the medical image the point whose location has been adjusted based on the type of caliper to be used to measure the object may include obtaining the medical image with respect to the object (S100), setting a measurement item and the type of caliper with respect to the object (S200), selecting a point in the medical image based on an external input (S300), adjusting a location of the selected point based on the type of caliper that has been set (S400), and indicating the adjusted point in the medical image (S500).

The obtaining of the medical image with respect to the object (S100) may include obtaining an ultrasound image with respect to the image by using ultrasound data transmitted/received to/from the object via a probe or the like. The ultrasound image with respect to the object may include an A mode image, a B mode image, an M mode image, a Doppler image, or an elastic image. Also, the obtaining of the medical image with respect to the object (S100) may include obtaining an x-ray image by using an x-ray photographing apparatus, obtaining a CT image by using a CT apparatus, or obtaining an MRI image with respect to the object by using an MRI photographing apparatus.

The measurement item may include at least one of a bi-parietal diameter (BPD), an occipitofrontal diameter (OFD), a head circumference (HC), a posterior ventricle diameter (Vp), a cerebellum diameter (CBLL), and a cisterna magna (CM). Also, the measurement item may include at least one of a crown lump length (CRL), an abdominal circumference (AC), a fetal trunk area (FTA), a femur length (FL), and an expected fetal weight (EFW), but it is not limited thereto.

The type of caliper may be determined so as to select at least one of an inner point, an outer point, and a middle point of a boundary of the object according to the measurement item.

The measurement item and the type of caliper with respect to the object may be automatically set based on a measurement item and a type of caliper corresponding to the measurement item, pre-stored in a storage device (not shown). For example, the measurement item and the type of caliper may be mapped with each other and pre-stored in the storage device, according to a standard of a picture archiving and communication system (PACS) or digital imaging and communications in medicine (DICOM). If a measurement part is selected by a user, the measurement item and the type of caliper with respect to the object may be set by loading the measurement item and the type of caliper from a system, such as the PACS or the like. The measurement item and the type of caliper may be mapped with each other as guidelines provided from the specific organizations described above.

In addition, the method of indicating in the medical image the point whose location has been adjusted based on the type of caliper to be used to measure the object, according to the present embodiment, may include setting the measurement item and the type of caliper according to an input applied by a user. For example, the user may directly set the type of caliper according to a measurement item. For example, the user may set the type of caliper as a middle point to a middle point of a boundary of the object, in order to measure a head circumference (HC).

The type of caliper may be set as an inner point to an outer point, a middle point to a middle point, an inner point to an inner point, an outer point to an outer point, according to the measurement item, but it is not limited thereto.

As described above, each type of caliper may be predetermined by being mapped with each measurement item by specific organizations, or, each type of caliper may be directly set by a user for each measurement item.

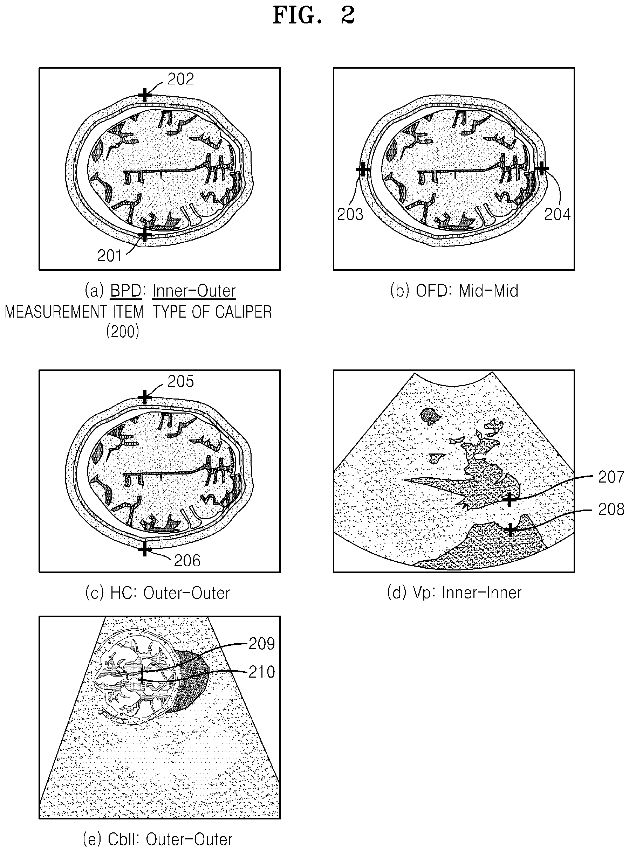

FIGS. 2A through 2E illustrate an example of a type of caliper that has been set according to a measurement item.

As illustrated in FIG. 2A, when a measurement item 200 is a BPD, the type of caliper may be set as an inner point to an outer point. In other words, the type of caliper to select at least one point 201 in the inside of a boundary of an object and at least one point 202 in the outside of the boundary of the object, may be set. Thus, the user may select only the inner point 201 and the outer point 202 of the boundary of the object, according to the type of caliper set as the inner point to the outer point, in order to measure the BPD.

Also, as illustrated in FIG. 2B, when the measurement item is an OFD, the type of caliper may be set as a middle point to a middle point. In other words, the type of caliper to select at least one middle point 203 of the boundary of the object and at least one middle point 204 which is different from the selected middle point 203, may be set. Thus, the user may select only the middle point 203 and another middle point 204 of the boundary of the object, according to the type of caliper set as the middle point to the middle point, in order to measure the OFD.

As illustrated in FIG. 2C, when the measurement item is a HC, the type of caliper may be set as an outer point to an outer point. In other words, the type of caliper to select at least one point 205 in the outside of the boundary of the object and at least one point 206 in the outside of the boundary, which is different from the selected point 205, may be set. Thus, the user may select only the outer point 205 and another outer point 206 of the boundary of the object, according to the type of caliper set as the outer point to the outer point, in order to measure the HC.

As illustrated in FIG. 2D, when the measurement item is a Vp, the type of caliper may be set as an inner point to an inner point. In other words, the type of caliper to select at least one point 207 in the inside of the boundary of the object and at least one point 208 in the inside of the boundary, which is different from the selected point 207, may be set. Thus, the user may select only the inner point 207 and another inner point 208 of the boundary of the object, according to the type of caliper set as the inner point to the inner point, in order to measure the Vp.

As illustrated in FIG. 2E, when the measurement item is a CBLL, the type of caliper may be set as an outer point to an outer point. In other words, the type of caliper to select at least one point 209 in the outside of the boundary of the object and at least one point 210 in the outside of the boundary, which is different from the selected point 209, may be set. Thus, the user may select only the outer point 209 and another outer point 210 of the boundary of the object, according to the type of caliper set as the outer point to the outer point, in order to measure the CBLL.

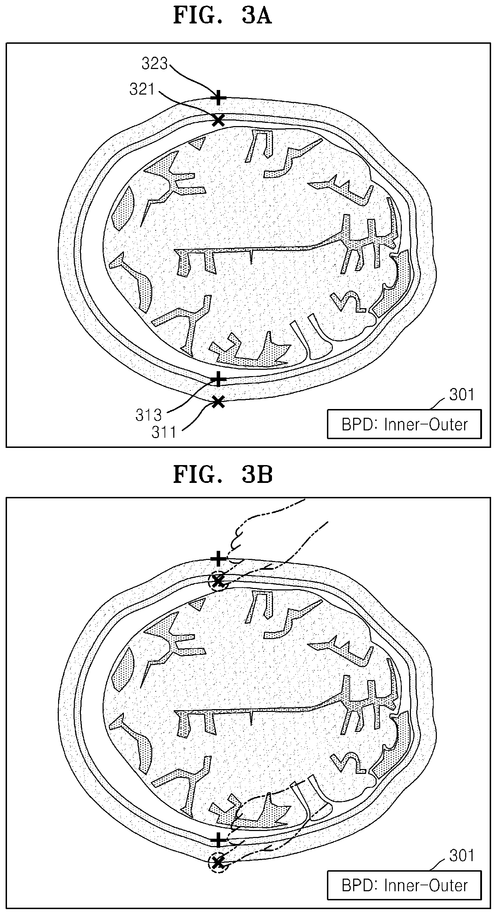

FIG. 3A illustrates an example of a point whose location has been adjusted in correspondence to a type of caliper, the point indicated in an ultrasound image, according to an embodiment of the present invention.

For example, when the measurement item is set as a BPD, information of a type of caliper 301 set in correspondence to the measurement item may be provided in a medical image. Thus, a user may select a point for the measurement according to the information of the type of caliper 301 that is provided. For example, when the type of caliper is set as an inner point to an outer point with respect to the measurement item, that is, the BPD, the user may select an inner point and an outer point of a boundary of an object to be measured. Therefore, the user may not need to memorize recommended measurement points according to measurement items, which may be provided as a guideline from a specific organization.

A predetermined point may be selected by shifting an identifier indicating the predetermined point (for example, an inner point, an outer point, or a middle point of a boundary) in a medical image and selecting any one point. For example, the identifier may be a cursor of an arrow shape or a finger shape, but it is not limited thereto. In other words, the user may move the cursor in the medical image and select a point to designate as a measurement point. The selected point may be referred to as a cursor point. A location of the cursor point may be changed. If the cursor point designated by the user as the measurement point is different from a recommended measurement point, it is difficult to accurately measure the object, and thus, the location of the cursor point may need to be changed to a location of the recommended measurement point. Such a recommended measurement point may be referred to as a caliper point.

FIG. 3B illustrates an example of indicating a point designated according to a touching input of a user by adjusting the point according to a type of caliper, according to an embodiment of the present invention.

A predetermined point (for example, an inner point, an outer point, and a middle point of a boundary) may be selected in a medical image indicated by an indication unit including a touch pad that enables a reception of a touching input, by a touching input applied from a user.

When the touch point selected by the user as a measurement point is different from a recommended measurement point, it is difficult to accurately measure an object, and thus, a location of the touch point may need to be changed to a location of the recommended measurement point. Such a recommended measurement point may be referred to as a caliper point. In other words, even if the user does not accurately touch the measurement point, accurate measurement may be achieved by changing the touch point to the recommended measurement point according to the type of caliper.

Referring again to the example described above, since the type of caliper is set as the inner point to the outer point with respect to the measurement item, that is, the BPD, a location of a first cursor point 311 may be changed to a location of an inner point 313 of a boundary of the object, which is a first caliper point, to be designated (or selected). Also, a location of a second cursor point 321 may be changed to a location of an outer point 323 of the boundary of the object, which is a second caliper point, to be designated.

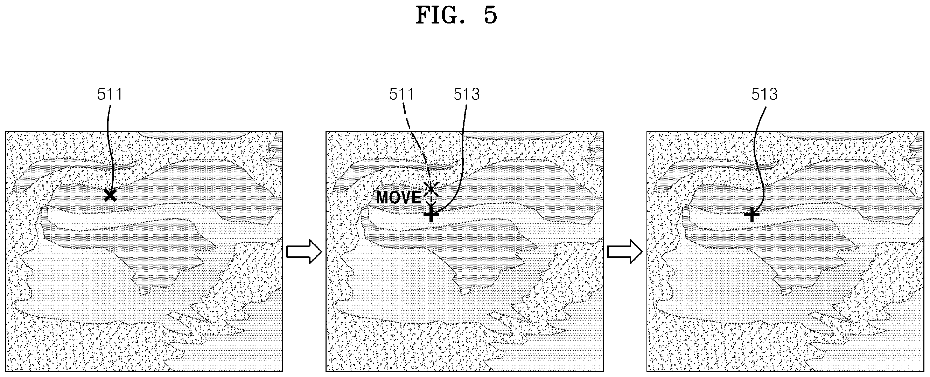

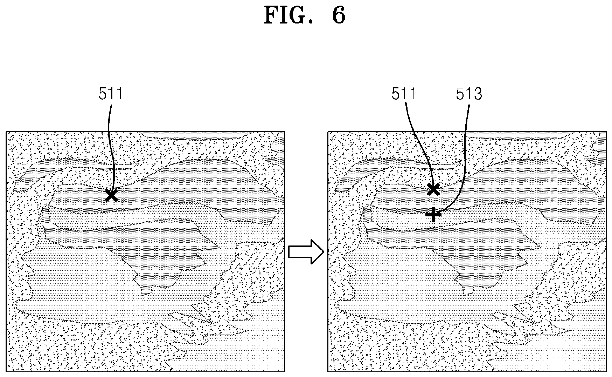

In addition, the change of the location of the point may include shifting the point 311 or 321 selected by the user to the caliper point for the measurement 313 or 323. Also, the point 311 or 321 selected by the user and the caliper point for the measurement 313 or 323 may be simultaneously indicated in the medical image. Aspects with respect to this matter will be described later by referring to FIGS. 5 and 6.

The selecting of the point in the medical image based on the external input (S300) according to the present embodiment may include determining an order of selecting the point according to the measurement item that has been set and selecting at least one point according to the determined order.

For example, when the measurement item is the BPD and the type of caliper is the inner point to the outer point, point designation time may be reduced because a point to be included as the inner point of the boundary of the object may be first designated (or selected) and a point to be included as the outer point of the boundary of the object may be later designated. For example, in the case in which an image of the inner portion of the boundary of the object is clear, while a image of the outer portion of the boundary of the object is not clear, in the medical image, the point of the inner portion of the boundary, which is relatively clear, may be first designated so that the overall time of designating the points may be reduced.

The order of selecting the point according to the measurement item that has been set may be pre-set according to the measurement item or may be directly set by the user. For example, the user may set the selection order such that a point located in an upper portion of the medical image may be selected first before a point located below the point located in the upper portion of the medical image, according to a measurement item. Also, the user may set the selection order such that a point located in a left portion of the medical image may be selected first before a point located in a right portion of the medical image, according to a measurement item.

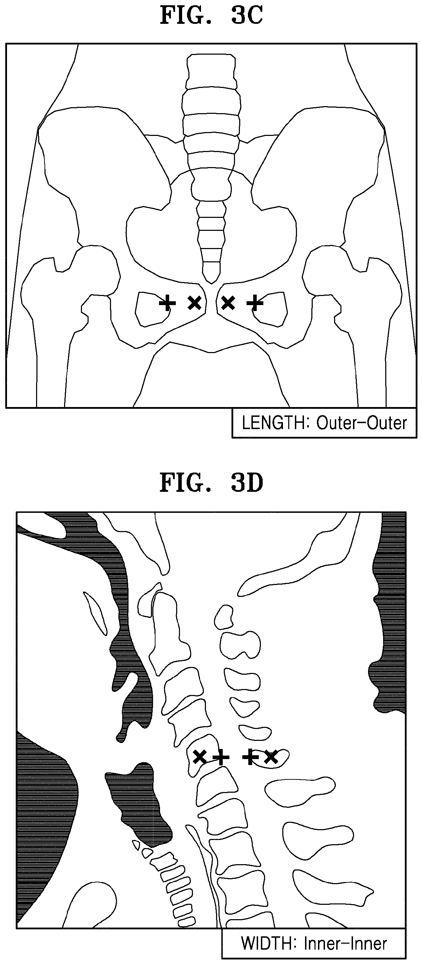

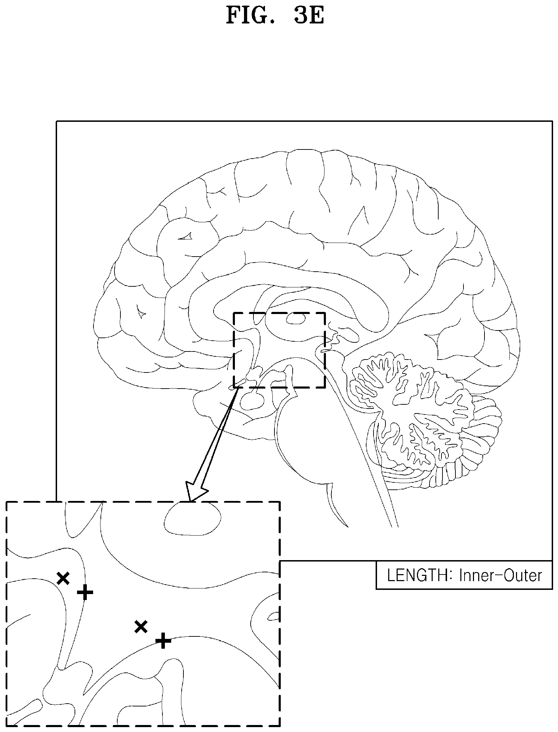

FIG. 3C illustrates an example of a point whose location has been adjusted in correspondence to a type of caliper, the point indicated in an x-ray image, according to an embodiment of the present invention. FIG. 3D illustrates an example of a point whose location has been adjusted in correspondence to a type of caliper, the point indicated in a CT image, according to an embodiment of the present invention. FIG. 3E illustrates an example of a point whose location has been adjusted in correspondence to a type of caliper, the point indicated in an MRI image, according to an embodiment of the present invention. The location adjusting and the indicating of the point in the x-ray image, the CT image, or the MRI image, according to the type of caliper, may be implemented similarly to the method described above.

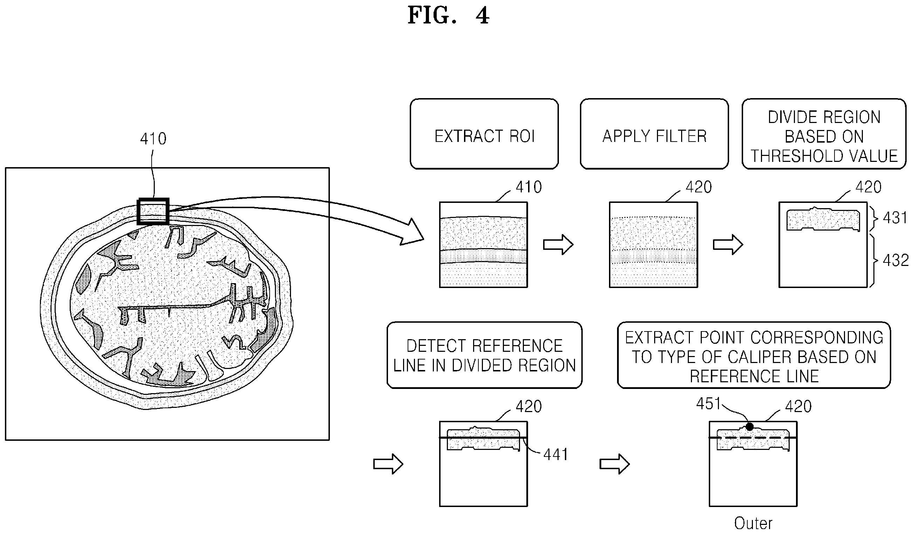

FIG. 4 illustrates a process of extracting a point corresponding to a type of caliper, according to an embodiment of the present invention.

The adjusting of the location of the selected point based on the type of caliper that has been set (S400) may include obtaining location information of the selected point, extracting a point adjacent to the selected point and corresponding to the type of caliper that has been set, and adjusting a location of the selected point according to location information of the extracted point.

A predetermined data processing method and an extraction algorithm may be used in the adjusting of the location of the selected point based on the type of caliper that has been set (S400).

For example, as illustrated in FIG. 4, a region of interest (ROI) 410 to designate a measurement point in a medical image with respect to an object may be selected. The predetermined data processing method may be applied with respect to medical data indicating the selected ROI 410 to perform medical data conversion. The predetermined data processing method may perform the conversion of the medical data indicating the selected ROI 410, by using a low pass filter, a max/min filter, a Sobel filter, and difference of Gaussian.

Also, the extraction algorithm may include line detection, cycle detection, and pitch detection.

Extracting of the location information of the point adjacent to the selected point and corresponding to the type of caliper that has been set may include extracting a predetermined region including the selected point from the medical image, extracting the point corresponding to the type of caliper that has been set based on a characteristic of the object corresponding to the extracted predetermined region, and obtaining the location information of the extracted point. The characteristic of the object may include an anatomical shape of the object or a brightness value of the medical image with respect to the object.

The extracting of the point adjacent to the selected point and corresponding to the type of caliper that has been set may include obtaining a region of interest 420 filtered by applying the earlier described data processing method with respect to the selected ROI 410.

In addition, the filtered region of interest 420 may be divided into two or more regions based on a threshold value. The threshold value may be a predetermined brightness value. For example, human bones have a higher brightness value than other organs in a medical image, and the brightness value of human bones may be used as the threshold value. The filtered region of interest 420 may be divided into two regions 431 and 432 based on the threshold value, as illustrated in FIG. 4.

A line detection algorithm may be applied to any of the divided two regions 431 and 432 to detect a reference line 441. For example, the reference line 441 may be a central line of a boundary of an object. In other words, the inside and the outside of the boundary of the object may be separated based on the reference line 441.

A caliper point according to the type of caliper may be extracted based on the detected reference line 441. The location information of the extracted point may be obtained based on a location of the ROI 410 in the medical image or a location of a pixel corresponding to the extracted point included in the ROI 410.

If the type of caliper is set as the inner point to the outer point with respect to the measurement item, that is, the BPD, as in the earlier described example, and an outer point of the boundary of the object is to be designated in the ROI 410 of FIG. 4, a point in the outside of the boundary of the object may be extracted based on the detected reference line 441. For example, an upper portion of the ROI 410 corresponds to the outside of the boundary, and thus, a point 451 located above the reference line 441 may be extracted as the outer point of the boundary of the object.

In addition, various data conversion methods may be used in the adjusting of the location of the selected point based on the type of caliper that has been set (S400). For example, the data conversion methods may include a random transform method, a fourier transform method, and a hough transform method.

The location information of the selected point may be obtained based on a location of a pixel of the point selected in the medical image.

The adjusting of the location of the selected point according to the location information of the extracted point may include comparing the location of the extracted point and the location of the selected point, and adjusting the location of the selected point so that the location of the selected point is changed to the location of the extracted point. If the user accurately designates the location of the selected point to correspond to the type of caliper, there is no need to change the location of the selected point. In other words, when the location of the extracted point and the location of the selected point are the same, the location of the selected point may not need to be changed.

However, when the location of the extracted point and the location of the selected point are different, the point for the measurement according to the type of caliper may be accurately designated by changing the location of the selected point.

FIG. 5 illustrates an example of a point whose location has been adjusted in correspondence to a type of caliper, the point indicated, according to an embodiment of the present invention.

The indicating of the adjusted point in the medical image may include indicating the adjusted point by shifting an identifier indicating the selected point.

As described above, the identifier may include a cursor of an arrow or finger shape. For example, if the measurement item is a Vp and the type of caliper is set as an inner point to an inner point, a cursor point 511 may be selected in the medical image by a user input. A location of a caliper point 513 corresponding to the type of caliper may be extracted by applying the various methods above described. As illustrated in FIG. 5, since locations of the cursor point 511 and the caliper point 513 are different from each other, the location of the cursor point 511 may be changed to the location of the caliper point 513. In other words, even if the cursor point 511 is selected by the user input, because the point corresponding to the type of caliper (for example, the caliper point) is an inner point 513 of the object, the location of the cursor point 511 is changed to a location of the inner point 513 so that the inner point 513 may be used for the measurement.

FIG. 6 illustrates an example of indicating a point whose location has been adjusted in correspondence to a type of caliper, according to another embodiment of the present invention.

The indicating of the adjusted point in the medical image may include indicating an identifier indicating the adjusted point, together with the identifier indicating the selected point.

As described above, the identifier may include the cursor of the arrow or finger shape. As in the example described above, when the measurement item is the Vp and the type of caliper is set as the inner point to the inner point, the cursor point 511 may be selected in the medical image by the user input. Also, the location of the caliper point 513 may be extracted by applying the various methods described above. When the locations of the cursor point 511 and the caliper point 513 are different from each other, the cursor point 511 and the caliper point 513 may be simultaneously indicated so that the caliper point 513 to be used for the measurement may be more clearly indicated.

In other words, even if the cursor point 511 is selected by the user input, because the point corresponding to the type of caliper (for example, the caliper point) is the inner point 513 of the object, the cursor point 511 and the inner point 513 may be simultaneously indicated so that the user may identify whether the point corresponding to the type of caliper is accurately designated.

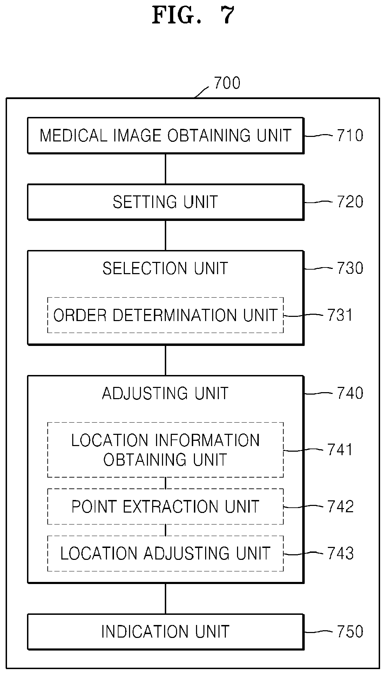

FIG. 7 is a block diagram of an apparatus for indicating in a medical image a point whose location has been adjusted based on a type of caliper to be used to measure an object, according to an embodiment of the present invention.

The apparatus 700 for indicating in the medical image the point whose location has been adjusted based on the type of caliper to be used to measure the object may include a medical image obtaining unit 710 that obtains the medical image with respect to the object, a setting unit 720 that sets a measurement item and the type of caliper with respect to the object, a selection unit 730 that selects a point in the medical image based on an external input, an adjusting unit 740 that adjusts a location of the selected point based the type of caliper that has been set, and an indication unit 750 that indicates the adjusted point in the medical image.

The measurement item may include at least one of a bi-parietal diameter (BPD), an occipitofrontal diameter (OFD), a femur length (FL), a head circumference (HC), a posterior ventricle diameter (Vp), a cerebellum diameter (CBLL), and a cisterna magna (CM). Also, the measurement item may include at least one of a crown lump length (CRL), an abdominal circumference (AC), a fetal trunk area (FTA), a femur length (FL), and an expected fetal weight (EFW), but it is not limited thereto.

The type of caliper may be determined so as to select at least one of an inner point, an outer point, and a middle point of a boundary of the object.

The selection unit 730 may further include an order determination unit 731 that determines an order of selecting the point according to the measurement item that has been set. The selection unit 730 may select at least one point according to the order determined by the order determination unit 731.

The adjusting unit 740 may include a location information obtaining unit 741 that obtains location information of the selected point, a point extraction unit 742 that extracts a point adjacent to the selected point and corresponding to the type of caliper that has been set, and a location adjusting unit 743 that adjusts a location of the selected point according to location information of the extracted point.

The point extraction unit 742 may extract a predetermined region including the selected point from the medical image, extract the point corresponding to the type of caliper that has been set based on a characteristic of the object corresponding to the extracted predetermined region, and obtain the location information of the extracted point. The characteristic of the object may include an anatomical shape of the object or a brightness value of the medical image with respect to the object.

The indication unit 750 may indicate the adjusted point by shifting an identifier indicating the selected point. Also, the indication unit 750 may indicate an identifier indicating the adjusted point, together with the identifier indicating the selected point.

The methods according to the embodiments of the present invention may apply the aspects with respect to the above described apparatus 700. Thus, aspects of the methods, which are identical with the aspects of the apparatus 700, are not described.

The above described embodiments of the present invention may be implemented as an executable program, and may be executed by a general-purpose digital computer that runs the program by using a computer-readable recording medium.

The computer-readable recording medium includes magnetic storage media (e.g., ROM, floppy disks, hard disks, etc.) and optical recording media (e.g., CD-ROMs, or DVDs).

It should be understood that the exemplary embodiments described herein should be considered in a descriptive sense only and not for purposes of limitation. Descriptions of features or aspects within each embodiment should typically be considered as available for other similar features or aspects in other embodiments.

## Claims

1. A method for processing a medical image, the method comprising:
obtaining the medical image with respect to an object including a part of a body;
setting, by an apparatus, a measurement item with respect to the part of the body to be measured from the medical image
and a type of caliper as an inner point to an outer point, a middle point to a middle point, an inner point to an inner point, or an outer point to an outer point, with respect to a boundary of the object, according to the set measurement item;
selecting, by the apparatus, a point in the medical image based on an external input;
adjusting, by the apparatus, a location of the selected point to correspond to the type of caliper that has been set, by:
obtaining location information of the selected point;
extracting a point adjacent to the selected point and corresponding to the type of caliper that has been set with respect to the boundary of the object, based on the obtained location information; and
adjusting a location of the selected point so that the location of the selected point is changed to a location of the extracted point; and
indicating, by the apparatus, the location-adjusted point in the medical image,
wherein extracting the point adjacent to the selected point and corresponding to the type of caliper that has been set comprises:
extracting a predetermined region including the selected point from the medical image;
dividing the extracted predetermined region into two or more regions based on a threshold value being a brightness value of the medical image with respect to the object;
detecting a central line of the boundary of the object as a reference line by applying a line detection algorithm to a region corresponding to the boundary of the object among the divided two or more regions;
extracting the point corresponding to the type of caliper that has been set, based on the detected reference line; and
obtaining the location information of the extracted point,
wherein the measurement item comprises at least one of a bi-parietal diameter (BPD), an occipitofrontal diameter (OFD), a femur length (FL), a head circumference (HC), a posterior ventricle diameter (Vp), cerebellum diameter (CBLL), a cisterna magna (CM), a crown lump length (CRL), abdominal circumference (AC), a fetal trunk area (FTA), or an expected fetal weight (EFW).

2. The method of claim 1, wherein the selecting of the point in the medical image based on the external input comprises determining an order of selecting the point according to the measurement item that has been set, and selecting at least one point according to the determined order.

3. The method of claim 1, wherein the indicating of the location-adjusted point in the medical image comprises indicating the adjusted point, by shifting an identifier indicating the selected point.

4. The method of claim 1, wherein the indicating of the location-adjusted point in the medical image comprises indicating an identifier indicating the location-adjusted point, together with an identifier indicating the selected point.

5. An apparatus for processing a medical image, the apparatus comprising:
an ultrasound image obtaining unit configured to obtain the medical image with respect to the object including a part of a body;
a setting unit configured to set a measurement item with respect to the part of the body to be measured from the medical image and a type of caliper as an inner point to an outer point, a middle point to a middle point, an inner point to an inner point, or an outer point to an outer point, with respect to a boundary of the object according to the set measurement item;
a selection unit configured to select a point in the medical image based on an external input;
an adjusting unit configured to adjust a location of the selected point to correspond to the type of caliper that has been set; and
an indication unit configured to indicate the adjusted point in the medical image,
wherein the adjusting unit comprises:
a location information obtaining unit configured to obtain location information of the selected point;
a point extraction unit configured to extract a point adjacent to the selected point and corresponding to the type of caliper that has been set with respect to the boundary of the object, based on the obtained location information; and
a location adjusting unit configured to adjust a location of the selected point so that the location of the selected point is changed to a location of the extracted point,
wherein the point extraction unit extracts a predetermined region including the selected point from the medical image, divides the extracted predetermined region into two or more regions based on a threshold value being a brightness value of the medical image with respect to the object, detects a central line of the boundary of the object as a reference line by applying a line detection algorithm to a region corresponding to the boundary of the object among the divided two or more regions, extracts the point corresponding to the type of caliper that has been set, based on the detected reference line, and obtains the location information of the extracted point,
wherein the measurement item comprises at least one of a bi-parietal diameter (BPD), an occipitofrontal diameter (OFD), a femur length (FL), a head circumference (HC), a posterior ventricle diameter (Vp), a cerebellum diameter (CBLL), a cisterna magna (CM), a crown lump length (CRL), abdominal circumference (AC), a fetal trunk area (FTA), or an expected fetal weight (EFW).

6. The apparatus of claim 5, wherein the selection unit further comprises an order determination unit configured to determine an order of selecting the point according to the measurement item that has been set, and the selection unit selects at least one point according to the determined order.

7. The apparatus of claim 5, wherein the indication unit indicates the adjusted point, by shifting an identifier indicating the selected point.

8. The apparatus of claim 5, wherein the indication unit indicates an identifier indicating the location-adjusted point, together with an identifier indicating the selected point.

9. A non-transitory computer-readable medium comprising instructions which, when executed by a computer, cause the computer to carry out the method of any of claims 1 through 4.

## Patentansprüche

1. Verfahren zur Verarbeitung einer medizinischen Abbildung, wobei das Verfahren Folgendes aufweist:
Erlangen der medizinischen Abbildung unter Bezugnahme auf ein Objekt, das einen Teil eines Körpers beinhaltet;
Festlegen, mittels einer Vorrichtung, eines Messelements unter Bezugnahme auf den Teil des Körpers, der von der medizinischen Abbildung zu messen ist;
und eine Art von Taster bzw. Zange als ein innerer Punkt zu einem äußeren Punkt, ein mittlerer Punkt zu einem mittleren Punkt, ein innerer Punkt zu einem inneren Punkt oder ein äußerer Punkt zu einem äußeren Punkt unter Bezugnahme auf eine Umrandung des Gegenstands, gemäß dem festgelegten Messelement;
Auswählen, mittels der Vorrichtung, einen Punkt in der medizinischen Abbildung basierend auf einer externen Eingabe;
Einstellen, mittels der Vorrichtung, einen Ort des ausgewählten Punkts, um mit der Art von Taster bzw. Zange, die festgelegt worden ist, zu korrespondieren, mittels:
Erlangen von Ortsinformationen des ausgewählten Punkts;
Extrahieren eines Punkts benachbart zu dem ausgewählten Punkt und entsprechend der Art von Taster bzw. Zange, die festgelegt worden ist, unter Bezugnahme auf die Umrandung des Gegenstands, basierend auf den erlangten Ortsinformationen; und
Einstellen eines Orts des ausgewählten Punkts, so dass der Ort des ausgewählten Punkts zu einem Ort des extrahierten Punkts geändert wird; und
Anzeigen, mittels der Vorrichtung, den bezüglich des Orts eingestellten Punkt in der medizinischen Abbildung,
wobei das Extrahieren des Punkts benachbart zu dem ausgewählten Punkt und entsprechend der Art des Tasters bzw. der Zange, die festgelegt worden ist, Folgendes aufweist:
Extrahieren eines vorbestimmten Bereichs, welcher den ausgewählten Punkt aus der medizinischen Abbildung beinhaltet;
Teilen des extrahierten, vorbestimmten Bereichs in zwei oder mehr Bereiche, basierend auf einem Grenzwert, bei dem es sich um einen Helligkeitswert des medizinischen Bilds unter Bezugnahme auf den Gegenstand handelt;
Detektieren einer mittleren Linie der Umrandung des Objekts als eine Referenzlinie durch Anwenden eines Liniendetektionsalgorithmus auf einen Bereich, der der Umrandung des Objekts unter den aufgeteilten zwei oder mehr Bereichen entspricht;
Extrahieren des Punkts entsprechend der Art von Taster bzw. Zange, die festgelegt worden ist, basierend auf der detektierten Referenzlinie; und
Erlangen der Ortsinformationen des extrahierten Punkts,
wobei das Messelement wenigstens eines eines biparietalen Durchmessers (BPD), eines okzipitofrontalen Durchmessers (OFD), einer Femurlänge (FL), eines Kopfumfangs (HC), eines hinteren Ventrikel-Durchmessers (VP), des Zerebellum-Durchmessers (CBLL), einer Cisterna Magna (CM), einer Scheitel-Steiß-Länge (CRL) eines Abdominal-Umfangs (AC), eines fetalen Rumpfbereichs (FTA) oder eines erwarteten Fötalgewichts (EFW) aufweist.

2. Verfahren nach Anspruch 1, wobei das Auswählen des Punkts in der medizinischen Abbildung basierend auf der externen Eingabe das Ermitteln einer Reihenfolge des Auswählens des Punkts gemäß dem Messelement, das festgelegt worden ist, und das Auswählen wenigstens eines Punkts gemäß der ermittelten Reihenfolge aufweist.

3. Verfahren nach Anspruch 1, wobei das Anzeigen des bezüglich seines Orts eingestellten Punkts in der medizinischen Abbildung das Anzeigen des eingestellten Punkts durch Verlagern eines Identifikators, der den ausgewählten Punkt anzeigt, beinhaltet.

4. Verfahren nach Anspruch 1, wobei das Anzeigen des bezüglich seines Orts eingestellten Punkts in der medizinischen Abbildung das Anzeigen eines Identifikators beinhaltet, der den bezüglich seines Orts eingestellten Punkt anzeigt, zusammen mit einem Identifikator, der den ausgewählten Punkt anzeigt.

5. Vorrichtung zur Verarbeitung einer medizinischen Abbildung, wobei die Vorrichtung Folgendes aufweist:
eine Ultraschallabbildungs-Erlangungseinheit, die dafür vorgesehen ist, die medizinische Abbildung unter Bezugnahme auf ein Objekt, das einen Teil eines Körpers beinhaltet, zu erlangen;
eine Festlegungseinheit, die dafür vorgesehen ist, ein Messelement unter Bezugnahme auf den Teil des Körpers, der von der medizinischen Abbildung zu messen ist, und eine Art von Taster bzw. Zange als einen inneren Punkt zu einem äußeren Punkt, einen mittleren Punkt zu einem mittleren Punkt, einem inneren Punkt zu einem inneren Punkt oder einem äußeren Punkt zu einem äußeren Punkt unter Bezugnahme auf eine Umrandung des Gegenstands gemäß dem festgelegten Messelement festzulegen;
eine Auswahleinheit, die dafür vorgesehen ist, einen Punkt in der medizinischen Abbildung basierend auf einer externen Eingabe auszuwählen;
eine Einstelleinheit, die dafür vorgesehen ist, einen Ort des ausgewählten Punkts, der mit der Art von Taster bzw. Zange, die festgelegt worden ist, korrespondieren soll, einzustellen; und
eine Anzeigeeinheit, die dafür vorgesehen ist, den eingestellten Punkt in der medizinischen Abbildung anzuzeigen;
wobei die Einstelleinheit Folgendes aufweist:
eine Ortsinformations-Erlangungseinheit, die dafür vorgesehen ist, Ortsinformationen des ausgewählten Punkts zu erlangen;
eine Punktextraktionseinheit, die dafür vorgesehen ist, einen Punkt benachbart zu dem ausgewählten Punkt und entsprechend der Art von Taster bzw. Zange, die festgelegt worden ist, unter Bezugnahme auf die Umrandung des Gegenstands, basierend auf den erlangten Ortsinformationen, zu extrahieren; und
eine Ortseinstelleinheit, die dafür vorgesehen ist, einen Ort des ausgewählten Punkts so einzustellen, dass der Ort des ausgewählten Punkts zu einem Ort des extrahierten Punkts geändert wird,
wobei die Punktextraktionseinheit einen vorbestimmten Bereich einschließlich des ausgewählten Punkts aus der medizinischen Abbildung extrahiert, den extrahierten Bereich in zwei oder mehr Bereiche basierend auf einem Grenzwert, bei dem es sich um einen Helligkeitswert der medizinischen Abbildung unter Bezugnahme auf den Gegenstand handelt, teilt, eine mittlere Linie der Umrandung des Objekts als eine Referenzlinie durch Anwenden eines Liniendetektionsalgorithmus auf einen Bereich, der der Umrandung des Objekts unter den aufgeteilten zwei oder mehr Bereichen entspricht, detektiert, den Punkt entsprechend der Art von Taster bzw. Zange, die festgelegt worden ist, basierend auf der detektierten Referenzlinie extrahiert und die Ortsinformationen des extrahierten Punkts erlangt,
wobei das Messelement wenigstens eines eines biparietalen Durchmessers (BPD), eines okzipitofrontalen Durchmessers (OFD), einer Femurlänge (FL), eines Kopfumfangs (HC), eines hinteren Ventrikel-Durchmessers (VP), des Zerebellum-Durchmessers (CBLL), einer Cisterna Magna (CM), einer Scheitel-Steiß-Länge (CRL) eines Abdominal-Umfangs (AC), eines fetalen Rumpfbereichs (FTA) oder eines erwarteten Fötalgewichts (EFW) aufweist.

6. Vorrichtung nach Anspruch 5, wobei die Auswahleinheit des Weiteren eine Reihenfolgen-Festlegeeinheit aufweist, die dafür vorgesehen ist, eine Reihenfolge des Auswählens des Punkts gemäß dem Messelement, das festgelegt worden ist, festzulegen, und wobei die Auswahleinheit wenigstens einen Punkt gemäß der festgelegten Reihenfolge auswählt.

7. Vorrichtung nach Anspruch 5, wobei die Anzeigeeinheit den eingestellten Punkt durch Verlagern eines Identifikators, der den ausgewählten Punkt anzeigt, anzeigt.

8. Vorrichtung nach Anspruch 5, wobei die Anzeigeeinheit einen Identifikator, der den bezüglich seines Orts eingestellten Punkt anzeigt, zusammen mit einem Identifikator, der den ausgewählten Punkt anzeigt, anzeigt.

9. Nicht flüchtiges, computerlesbares Medium, das Befehle aufweist, die, wenn sie von einem Computer ausgeführt werden, den Computer dazu bringen, das Verfahren nach einem der Ansprüche 1 bis 4 auszuführen.

## Revendications

1. Procédé de traitement d'une image médicale, le procédé comprenant :
l'obtention de l'image médicale par rapport à un objet comprenant une partie d'un corps ;
le réglage, par un appareil, d'un élément de mesure par rapport à la partie du corps à mesurer à partir de l'image médicale
et un type d'étrier comme un point intérieur par rapport à un point extérieur, un point central par rapport à un point central, un point intérieur par rapport à un point intérieur, ou un point extérieur par rapport à un point extérieur, par rapport à une limite de l'objet, selon l'élément de mesure défini ;
la sélection, par l'appareil, d'un point dans l'image médicale sur la base d'une entrée externe ;
le réglage, par l'appareil, d'un emplacement du point sélectionné pour qu'il corresponde au type d'étrier qui a été réglé, par :
l'obtention des informations de localisation du point sélectionné ;
l'extraction d'un point adjacent au point sélectionné et correspondant au type d'étrier qui a été réglé par rapport à la limite de l'objet, sur la base des informations de localisation obtenues ; et
l'ajustement d'un emplacement du point sélectionné de sorte que l'emplacement du point sélectionné soit modifié en un emplacement du point extrait ; et
l'indication, par l'appareil, du point à emplacement ajusté dans l'image médicale,
dans lequel l'extraction du point adjacent au point sélectionné et correspondant au type d'étrier qui a été réglé comprend :
l'extraction d'une région prédéterminée comprenant le point sélectionné de l'image médicale ;
la division de la région prédéterminée extraite en deux ou plusieurs régions sur la base d'une valeur seuil qui est une valeur de luminosité de l'image médicale par rapport à l'objet ;
la détection d'une ligne centrale de la limite de l'objet comme ligne de référence en appliquant un algorithme de détection de ligne à une région correspondant à la limite de l'objet parmi les deux ou plusieurs régions divisées ;
l'extraction du point correspondant au type d'étrier qui a été réglé, sur la base de la ligne de référence détectée ; et
l'obtention des informations de localisation du point extrait,
dans lequel l'élément de mesure comprend au moins un des éléments suivants : un diamètre bipariétal (BPD), un diamètre occipito-frontal (OFD), une longueur de fémur (FL), une circonférence de tête (HC), un diamètre du ventricule postérieur (Vp), un diamètre du cervelet (CBLL), une cisterna magna (CM), une longueur de la masse de la couronne (CRL), une circonférence abdominale (AC), une zone du tronc fœtal (FTA), ou un poids fœtal attendu (EFW).

2. Procédé selon la revendication 1, dans lequel la sélection du point dans l'image médicale sur la base de l'entrée externe comprend la détermination d'un ordre de sélection du point selon l'élément de mesure qui a été défini, et la sélection d'au moins un point selon l'ordre déterminé.

3. Procédé selon la revendication 1, dans lequel l'indication du point à emplacement ajusté dans l'image médicale comprend l'indication du point ajusté, en décalant un identificateur indiquant le point sélectionné.

4. Procédé selon la revendication 1, dans lequel l'indication du point à emplacement ajusté dans l'image médicale comprend l'indication d'un identificateur indiquant le point à emplacement ajusté, ainsi qu'un identificateur indiquant le point sélectionné.

5. Appareil de traitement d'une image médicale, l'appareil comprenant :
une unité d'obtention d'images ultrasonores configurée pour obtenir l'image médicale par rapport à l'objet comprenant une partie d'un corps ;
une unité de réglage configurée pour régler un élément de mesure par rapport à la partie du corps à mesurer à partir de l'image médicale et un type d'étrier comme un point intérieur par rapport à un point extérieur, un point central par rapport à un point central, un point intérieur par rapport à un point intérieur, ou un point extérieur par rapport à un point extérieur, par rapport à une limite de l'objet selon l'élément de mesure défini ;
une unité de sélection configurée pour sélectionner un point dans l'image médicale sur la base d'une entrée externe ;
une unité de réglage configurée pour régler un emplacement du point sélectionné pour qu'il corresponde au type d'étrier qui a été réglé ; et
une unité d'indication configurée pour indiquer le point ajusté dans l'image médicale,
dans lequel l'unité de réglage comprend :
une unité d'obtention d'informations de localisation configurée pour obtenir des informations de localisation du point sélectionné ;
une unité d'extraction de point configurée pour extraire un point adjacent au point sélectionné et correspondant au type d'étrier qui a été défini par rapport à la limite de l'objet, sur la base des informations de localisation obtenues ; et
une unité d'ajustement de la localisation configurée pour ajuster un emplacement du point sélectionné de sorte que l'emplacement du point sélectionné soit modifié en un emplacement du point extrait,
dans lequel l'unité d'extraction de point extrait une région prédéterminée comprenant le point sélectionné de l'image médicale, divise la région prédéterminée extraite en deux ou plusieurs régions sur la base d'une valeur seuil qui est une valeur de luminosité de l'imagerie médicale par rapport à l'objet, détecte une ligne centrale de la limite de l'objet comme ligne de référence en appliquant un algorithme de détection de ligne à une région correspondant à la limite de l'objet parmi les deux ou plusieurs régions divisées, extrait le point correspondant au type d'étrier qui a été réglé, sur la base de la ligne de référence détectée, et obtient les informations de localisation du point extrait,
dans lequel l'élément de mesure comprend au moins un des éléments suivants : un diamètre bipariétal (BPD), un diamètre occipito-frontal (OFD), une longueur de fémur (FL), une circonférence de tête (HC), un diamètre du ventricule postérieur (Vp), un diamètre du cervelet (CBLL), une cisterna magna (CM), une longueur de la masse de la couronne (CRL), une circonférence abdominale (AC), une zone du tronc fœtal (FTA), ou un poids fœtal attendu (EFW).

6. Appareil selon la revendication 5, dans lequel l'unité de sélection comprend en outre une unité de détermination d'ordre configurée pour déterminer un ordre de sélection du point selon l'élément de mesure qui a été défini, et l'unité de sélection sélectionne au moins un point selon l'ordre déterminé.

7. Appareil selon la revendication 5, dans lequel l'unité d'indication indique le point ajusté, en décalant un identificateur indiquant le point sélectionné.

8. Appareil selon la revendication 5, dans lequel l'unité d'indication indique un identificateur indiquant le point à emplacement ajusté, ainsi qu'un identificateur indiquant le point sélectionné.

9. Support non transitoire lisible par ordinateur comprenant des instructions qui, lorsqu'elles sont exécutées par un ordinateur, amènent l'ordinateur à exécuter le procédé selon l'une quelconque des revendications 1 à 4.
